# EUROPEAN PATENT APPLICATION

(11) **EP 4 671 266 A1**
(43) Date of publication of application: **31.12.2025**
(21) Application number: 25766470.6
(22) Date of filing: 23.04.2025
(51) Int. Cl.: C07K 14/405, C07K 19/00, C12N 15/31, C12N 15/62, C12N 15/861, C12N 15/864, A61K 48/00, A61K 38/16, A61P 9/10, A61P 27/02

(54) **NEW PHOTOSENSITIVE CHANNEL PROTEIN VR2.0 AND USE THEREOF**

(30) Priority: 05.07.2024 CN 202410900732
(71) Applicant: ZHONGMOU THERAPEUTICS CO., LTD., Wuhan, Hubei 430074 (CN)
(72) Inventor: SHEN, Yin, Wuhan Hubei 430074 (CN)
(74) Representative: Lavoix
(86) International application number: PCT/CN2025/090656
(87) International publication number: WO 2025/209599

(57) **Abstract**

The application provides a novel light-sensitive channel protein VR2.0 and uses thereof. The light-sensitive channel protein VR2.0 series provided in this application demonstrates profound therapeutic effects in the treatment of retinal photoreceptor cell degenerative diseases. This novel light-sensitive channel protein exhibits enhanced photosensitivity, fast kinetics, and reduced side effects. Under identical photostimulation conditions, VR2.0 demonstrates faster response frequencies while maintaining stable photocurrent signals even during high-frequency stimulation. This application provides a better optogenetic therapeutic option for retinal photoreceptor cell degenerative diseases, and has significant application and promotion value.

## Description

### TECHNICAL FIELD

This application belongs to the field of biomedicine, specifically, it relates to a novel light-sensitive channel protein VR2.0 and uses thereof.

### BACKGROUND

Retinal photoreceptor cell degenerative disease is a type of degenerative disease primarily characterized by progressive functional loss of photoreceptor cells and retinal pigment epithelial cells. This type of disease is mainly caused by genetic mutations or dysfunction of retinal pigment epithelial cells (RPE cells), with typical examples including retinitis pigmentosa (RP) and age-related macular degeneration (AMD), both of which are major and intractable blinding eye diseases. The prevalence of hereditary photoreceptor degenerative disorder is about 1/3500-1/4000. Currently, there are 400000 patients with retinitis pigmentosa in China and over 1.5 million patients worldwide. The prevalence of secondary retinal photoreceptor degenerative diseases caused by factors such as drug administration and diseases is also increasing.

Due to the irreversible apoptosis of photoreceptor cells in retinal photoreceptor cell degenerative disease and the high genetic heterogeneity of most such diseases, the treatment of related diseases has become extremely challenging. Current therapeutic approaches primarily include stem cell transplantation, gene therapy, retinal prosthesis implantation, and optogenetic therapy. Among them, optogenetic therapy leverages the structural integrity of residual cells in retinal degenerative diseases by using recombinant adeno-associated virus (AAV) as a vector to target the expression of photosensitive proteins in cone cells for early stage of degenerative diseases, bipolar cells, or ganglion cells for mid-to-late-stage degenerative diseases, thereby restoring retinal photosensitivity. In addition, stem cell transplantation, virtual reality systems, and holographic imaging technology can be combined to restore visual function.

Optogenetics-based therapy, as an ophthalmic treatment strategy that is not reliant on the correction of specific gene mutations and enables light responsiveness at the single-cell level, has great potential for the treatment of retinal photoreceptor degenerative disorders. The photosensitive proteins employed in optogenetic visual restoration strategies are mainly divided into two categories: microbial photosensitive proteins and mammalian photosensitive proteins. Microbial photosensitive proteins typically exhibit fast kinetic, but low photosensitivity, such as the cation-permeable photosensitive protein Channelrhodopsin-2 (ChR2), which was the first photosensitive protein applied for visual function restoration. Endogenous mammalian photosensitive proteins belong to the G Protein Coupled Receptors (GPCRs) family, with seven transmembrane alpha-helix domains. They typically exhibit high photosensitivity but lack sufficient kinetic performance, such as rhodopsin derived from rod photoreceptor cells (Rod). Currently, available optogenetic tools (i.e., photosensitive proteins) used for visual restoration fail to adequately meet the dual requirements of high light sensitivity and fast photoresponse kinetics. They also present issues such as ion selectivity discrepancies and side effects like intracellular acidification. Therefore, it is necessary to develop a class of photosensitive proteins with enhanced light sensitivity, faster photoresponse kinetics, minimal side effects, and the ability to maintain stable current signals under high-frequency light stimulation. Coupled with more efficient gene delivery vectors targeting retinal cells, optogenetic therapy can be better used in the treatment of retinal photoreceptor cell degenerative diseases.

### SUMMARY

To address the limitations of existing technology, this application provides a novel light-sensitive channel protein VR2.0 and uses thereof.

Specifically, this application involves the following aspects:
1. A light-sensitive channel protein comprising any one of the following PsCatCh variants:
   (1) a protein obtained by truncating 1-33 amino acids at the N-terminus of the reference protein shown in SEQ ID NO.1;
   (2) a protein obtained by truncating 1-29 amino acids at the C-terminus of the reference protein shown in SEQ ID NO.1;
   (3) a protein derived from mutations introduced around the retinal binding site of the reference protein shown in SEQ ID NO.1;
   (4) a combination of any two or three of the above (1), (2), and (3); or
   (5) a protein having more than 90%, 91%, 92%, 93%, 94%, 95%, 96%, 97%, 98%, or 99% sequence identity with the proteins described in (1), (2), (3), or (4).
2. The light-sensitive channel protein according to item 1, wherein the light-sensitive channel protein comprises a protein obtained by truncating 12-26 amino acids at the N-terminus of the reference protein shown in SEQ ID NO.1.
3. The light-sensitive channel protein according to item 2, wherein the light-sensitive channel protein comprises a protein obtained by truncating 17-21 amino acids at the N-terminus of the reference protein shown in SEQ ID NO.1.
4. The light-sensitive channel protein according to any one of items 1-3, wherein the light-sensitive channel protein comprises a protein obtained by truncating 9-23 amino acids at the C-terminus of the reference protein shown in SEQ ID NO.1.
5. The light-sensitive channel protein according to item 4, wherein the light-sensitive channel protein comprises a protein obtained by truncating 9-18 amino acids at the C-terminus of the reference protein shown in SEQ ID NO.1.
6. The light-sensitive channel protein according to item 1, wherein the light-sensitive channel protein comprises a protein obtained by truncating 12-21 amino acids at the N-terminus and 13-23 amino acids at the C-terminus of the reference protein shown in SEQ ID NO.1.
7. The light-sensitive channel protein according to any one of items 1-6, wherein the light-sensitive channel protein comprises a protein obtained by introducing the following mutations into the reference protein shown in SEQ ID NO.1 or the PsCatCh variant thereof: substitution of E at position 66 with D, and/or substitution of C at position 165 with L.
8. The light-sensitive channel protein according to any one of items 1-7, wherein the amino acid sequence of the light-sensitive channel protein is shown in SEQ ID NO.8, SEQ ID NO.9, SEQ ID NO.13, SEQ ID NO.14, SEQ ID NO.15, SEQ ID NO.16, SEQ ID NO.19, SEQ ID NO.20, SEQ ID NO.22, SEQ ID NO.23, SEQ ID NO.24, SEQ ID NO.25, SEQ ID NO.26, SEQ ID NO.28, SEQ ID NO.29, SEQ ID NO.31, SEQ ID NO.32, SEQ ID NO.33, SEQ ID NO.34, SEQ ID NO.35, or SEQ ID NO.36, or
   has more than 90%, 91%, 92%, 93%, 94%, 95%, 96%, 97%, 98%, or 99% sequence identity with SEQ ID NO.8, SEQ ID NO.9, SEQ ID NO.13, SEQ ID NO.14, SEQ ID NO.15, SEQ ID NO.16, SEQ ID NO.19, SEQ ID NO.20, SEQ ID NO.22, SEQ ID NO.23, SEQ ID NO.24, SEQ ID NO.25, SEQ ID NO.26, SEQ ID NO.28, SEQ ID NO.29, SEQ ID NO.31, SEQ ID NO.32, SEQ ID NO.33, SEQ ID NO.34, SEQ ID NO.35, or SEQ ID NO.36.
9. The light-sensitive channel protein according to any one of items 1-8, wherein the light-sensitive channel protein further comprises an LR signal peptide fused to the N-terminus of the PsCatCh variant, and/or
   a T peptide linked to the C-terminus of the PsCatCh variant and a E peptide linked to the T peptide,
   wherein the amino acid sequence of the LR signal peptide is shown in SEQ ID NO.2, the amino acid sequence of the T peptide is shown in SEQ ID NO.4, and the amino acid sequence of the E peptide is shown in SEQ ID NO.5.
10. A nucleic acid molecule comprising a nucleotide sequence encoding the light-sensitive channel protein according to any one of items 1-9.
11. A vector comprising the nucleic acid molecule according to item 10.
12. A recombinant virus comprising the nucleic acid molecule according to item 10 or the vector according to item 11.
13. The recombinant virus according to item 12, wherein the recombinant virus is a recombinant adeno-associated virus.
14. A pharmaceutical composition comprising the light-sensitive channel protein according to any one of items 1-9, the nucleic acid molecule according to item 10, the vector according to item 11, or the recombinant virus according to item 12 or 13, and a pharmaceutically acceptable carrier.
15. Use of the light-sensitive channel protein according to any one of items 1-9 in the preparation of a medicament for treating retinal photoreceptor cell degenerative diseases.
16. The use according to item 15, wherein the retinal photoreceptor cell degenerative diseases comprise retinitis pigmentosa (RP), age-related macular degeneration (AMD), cone-rod dystrophy, and Leber congenital amaurosis (LCA).

### Advantages and beneficial effects of the present application:

The light-sensitive channel protein VR2.0 series provided in this application demonstrates definitive therapeutic effect in the treatment of retinal photoreceptor cell degenerative diseases. This novel light-sensitive channel protein combines high sensitivity and fast kinetics, maintaining stable photocurrent signals under high-frequency stimulation while exhibiting faster response frequencies under identical light stimulation conditions. This application provides a new and superior alternative for developing optogenetic therapies for retinal photoreceptor cell degenerative disease, expands the scope of clinical optogenetic therapy for diseases, and has significant application and promotion value.

### BRIEF DESCRIPTION OF THE DRAWINGS

Fig. 1 shows the simulated three-dimensional structural diagrams of the photosensitive proteins PsCatCh (1-305aa), PsCatCh 2.0 (1-343aa), and the representative mutant PsCatCh 2.0 e26.
Fig. 2 shows a schematic diagram of the amino acid structure of the photosensitive protein VR2.0 series modified from the photosensitive protein PsCatCh.
Fig. 3 shows the photoresponse current intensity signals of the mutant variants within the photosensitive protein VR2.0 series.
Fig. 4 shows the waveform of the photoresponse electrophysiological signals of some mutants of the photosensitive protein VR2.0 series at the Xenopus laevis oocyte level.
Fig. 5 shows the permeability of Na⁺ and H⁺ ions of some mutants of the photosensitive protein VR2.0 series.
Fig. 6 shows the core vector pAAV-CMV-VR2.0-EYFP carrying expression cassettes of some mutants of the photosensitive protein VR2.0 series.
Fig. 7 shows the patch-clamp recordings of light-evoked currents in HEK293T cells expressing some mutants of the photosensitive protein VR2.0 series, with PsCatCh2.0 serving as the control group, reflecting the photosensitivity and response frequency of photosensitive proteins to light stimulation. Fig. 7A: current graph of VR2.0 under 470 nm wavelength light (intensity: 1.66×10¹⁵ photons/cm²s) at stimulation frequencies of 2Hz, 4Hz, 8Hz, 16Hz, and 32Hz; Fig. 7B: current graph of VR2.0 under 470 nm wavelength light (intensity: 1.66×10¹⁵ photons/cm²s) during a 1s stimulation.
Fig. 8 shows the whole-cell patch-clamp recordings of photocurrent amplitudes for some mutants of the photosensitive protein VR2.0 series and the control group PsCatCh2.0 expressed in HEK293T cells under 470 nm light stimulation at varying light intensities, where "photons/cm²s" is the unit of light intensity.
Fig. 9 shows the visual evoked potentials of C57BL/6J mice, *rd1* mice treated with intravitreal injection of rAAV2-CMV-VR2.0-EYFP, and untreated littermate *rd1* mice. Fig. 9A: representative waveforms of the visual evoked potentials of the above mice; Fig. 9B: statistical histogram of N1 wave amplitudes of visual evoked potential of the aforementioned mice. Among them, P<0.001 indicates a significant difference.
Fig. 10 shows the phototaxis avoidance responses of C57BL/6J mice, *rd1* mice treated with intravitreal injection of rAAV2-CMV-VR2.0-EYFP, and untreated littermate *rd1* mice in the light-dark box test. Fig. 10A: schematic diagram of the light-dark box experiment; Fig. 10B: statistical histogram of the activity time of the mice in the light box. A statistically significant difference was observed (P<0.05).
Fig. 11 shows the optomotor responses of C57BL/6J mice, *rdl* mice treated with intravitreal injection of rAAV2-CMV-VR2.0-EYFP, and untreated littermate *rd1* mice. Fig. 11A: schematic diagram of the optomotor response testing setup; Fig. 11B: statistical bar chart of visual acuity of the above mice. Statistical significance was noted (P<0.05).

### DETAILED DESCRIPTION OF THE APPLICATION

The following will further illustrate the present application in conjunction with the embodiments. It should be understood that the embodiments are only used to further illustrate and clarify the present application, and are not intended to limit the present application.

Unless otherwise defined, the technical and scientific terms used in this description have the same meanings as those commonly understood by those skilled in the art. Although similar or identical methods and materials can be applied in experiments or practical applications, this application still describes the materials and methods in the following text. In case of conflict, this description, including its definitions, shall prevail. Additionally, materials, methods, and examples are for illustration purposes only and are not restrictive. The following provides further explanation of the present application in conjunction with specific embodiments, but is not intended to limit the scope of the present application.

As used herein, "light-sensitive channel protein" and "light-sensitive protein" may be used interchangeably, referring to a class of proteins located on the cell membrane capable of sensing light stimulation and producing specific effects such as changing the open state of ion channels. These proteins can be divided into activating and inhibitory types, which induce neuronal excitation or inhibition.

The PsCatCh variant in this application is engineered based on the *Platymonas subcordiformis* channelrhodopsin (PsChR).

### Light-sensitive channel protein

This application provides a light-sensitive channel protein comprising any one of the following PsCatCh variants:
(1) a protein obtained by truncating 1-33 amino acids at the N-terminus of the reference protein shown in SEQ ID NO.1;
(2) a protein obtained by truncating 1-29 amino acids at the C-terminus of the reference protein shown in SEQ ID NO.1;
(3) a protein derived from mutation introduced around the retinal binding site of the reference protein shown in SEQ ID NO.1;
(4) a combination of any two or three of the above (1), (2), and (3); or
(5) a protein having at least 60% sequence identity with (1), (2), (3), or (4), for example a protein having more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 94%, 96%, 98%, 91%, 92%, 93%, 94%, 95%, 96%, 97%, 98%, or 99% sequence identity with (1), (2), (3), or (4).

In a specific embodiment, the PsCatCh variant is a protein obtained by truncating 1-33 amino acids at the N-terminus of the reference protein shown in SEQ ID NO.1. Among them, 1-33 amino acids may be, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or 33 amino acids.

In a specific embodiment, the PsCatCh variant is a protein obtained by truncating 12-26 amino acids at the N-terminus of the reference protein shown in SEQ ID NO.1.

In a specific embodiment, the PsCatCh variant is a protein obtained by truncating 17-21 amino acids at the N-terminus of the reference protein shown in SEQ ID NO.1.

In a specific embodiment, the amino acid sequence of the PsCatCh variant is shown in any one of SEQ ID NO.8, SEQ ID NO.9, SEQ ID NO.10, SEQ ID NO.11, SEQ ID NO.12, or SEQ ID NO.13, or has more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 94%, 96%, 98%, 91%, 92%, 93%, 94%, 95%, 96%, 97%, 98%, or 99% sequence identity with any one of SEQ ID NO.8, SEQ ID NO.9, SEQ ID NO.10, SEQ ID NO.11, SEQ ID NO.12, or SEQ ID NO.13.

In a specific embodiment, the PsCatCh variant is a protein obtained by truncating 1-29 amino acids at the C-terminus of the reference protein shown in SEQ ID NO.1. Among them, 1-29 amino acids may be, for example, 1, 2, 3, 4, 5, 6, 7, 8, 9, 10, 11, 12, 13, 14, 15, 16, 17, 18, 19, 20, 21, 22, 23, 24, 25, 26, 27, 28, or 29 amino acids.

In a specific embodiment, the PsCatCh variant is a protein obtained by truncating 9-23 amino acids at the C-terminus of the reference protein shown in SEQ ID NO.1.

In a specific embodiment, the PsCatCh variant is a protein obtained by truncating 9-18 amino acids at the C-terminus of the reference protein shown in SEQ ID NO.1.

In a specific embodiment, the amino acid sequence of the PsCatCh variant is shown in any one of SEQ ID NO.14, SEQ ID NO.15, SEQ ID NO.16, SEQ ID NO.17, SEQ ID NO.18, or SEQ ID NO.19, or has more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 94%, 96%, 98%, 91%, 92%, 93%, 94%, 95%, 96%, 97%, 98%, or 99% sequence identity with any one of SEQ ID NO.14, SEQ ID NO.15, SEQ ID NO.16, SEQ ID NO.17, SEQ ID NO.18, or SEQ ID NO.19.

In a specific embodiment, the PsCatCh variant is a protein obtained by truncating 1-33 amino acids at the N-terminus and 1-29 amino acids at the C-terminus of the reference protein shown in SEQ ID NO.1.

In a specific embodiment, the PsCatCh variant is a protein obtained by truncating 12-21 amino acids at the N-terminus and 13-23 amino acids at the C-terminus of the reference protein shown in SEQ ID NO.1.

In a specific embodiment, the amino acid sequence of the PsCatCh variant is shown in any one of SEQ ID NO.29, SEQ ID NO.30, SEQ ID NO.31, SEQ ID NO.32, SEQ ID NO.33, SEQ ID NO.34, SEQ ID NO.35, or SEQ ID NO.36, or has more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 94%, 96%, 98%, 91%, 92%, 93%, 94%, 95%, 96%, 97%, 98%, or 99% sequence identity with any one of SEQ ID NO.29, SEQ ID NO.30, SEQ ID NO.31, SEQ ID NO.32, SEQ ID NO.33, SEQ ID NO.34, SEQ ID NO.35, or SEQ ID NO.36.

In a specific embodiment, the PsCatCh variant is a protein obtained by introducing the following mutations into the reference protein shown in SEQ ID NO.1 or the PsCatCh variant thereof: substitution of E at position 66 with D, and/or substitution of C at position 165 with L. Wherein regarding the point mutations in the PsCatCh variant, positions 66 and 165 correspond to those in the reference protein shown in SEQ ID NO.1.

In a specific embodiment, the amino acid sequence of the PsCatCh variant is shown in any one of SEQ ID NO.20, SEQ ID NO.21, SEQ ID NO.22, SEQ ID NO.23, SEQ ID NO.24, SEQ ID NO.25, SEQ ID NO.26, SEQ ID NO.27, or SEQ ID NO.28, or has more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 94%, 96%, 98%, 91%, 92%, 93%, 94%, 95%, 96%, 97%, 98%, or 99% sequence identity with any one of SEQ ID NO.20, SEQ ID NO.21, SEQ ID NO.22, SEQ ID NO.23, SEQ ID NO.24, SEQ ID NO.25, SEQ ID NO.26, SEQ ID NO.27, or SEQ ID NO.28.

In a specific embodiment, the light-sensitive channel protein further comprises an LR signal peptide fused to the N-terminus of the PsCatCh variant.

In a specific embodiment, the amino acid sequence of the LR signal peptide is shown in SEQ ID NO.2.

In a specific embodiment, the light-sensitive channel protein further comprises a T peptide connected to the C-terminus of the PsCatCh variant to increase cell membrane expression efficiency, and an endoplasmic reticulum export signal sequence E peptide connected to the T peptide.

In a specific embodiment, the amino acid sequence of the T peptide is shown in SEQ ID NO.4. In a specific embodiment, the amino acid sequence of the E peptide is shown in SEQ ID NO.5.

Those skilled in the art can understand that between any two of the PsCatCh variant, T peptide, and E peptide, there may also be a linker peptide sequence composed of a small number of amino acids that does not affect the function of the light-sensitive channel protein.

In a specific embodiment, the amino acid sequence of the linker peptide is shown in SEQ ID NO.37.

### Nucleic acid molecules, vectors, recombinant viruses

This application provides a nucleic acid molecule comprising a nucleotide sequence encoding any of the aforementioned light-sensitive channel proteins.

In some embodiments, the nucleic acid molecule is an engineered DNA molecule. In some embodiments, the DNA molecule can replicate and/or be expressed in cells. In some embodiments, the DNA molecule can replicate and/or be expressed in eukaryotic cells. In some embodiments, the DNA molecule may replicate and/or be expressed in prokaryotic cells. In some embodiments, the DNA molecule can be expressed in eukaryotic cells and replicated in prokaryotic cells. Therefore, in addition to the nucleotide sequence encoding the light-sensitive channel protein, the DNA molecule also comprises genetic manipulation or regulatory elements for replication and/or expression in prokaryotic and/or eukaryotic cells. In some embodiments, the eukaryotic cells are human retinal photoreceptor cells. In some embodiments, the eukaryotic cells are human cone cells. In some embodiments, the eukaryotic cells are bipolar cells or ganglion cells.

This application provides a vector comprising the aforementioned nucleic acid molecule.

In some embodiments, the vector is a DNA plasmid. As used herein, the term "DNA plasmid" refers to a plasmid composed of double stranded DNA molecules. In some embodiments, the "plasmid" is a circular DNA molecule. In some embodiments, the "plasmid" may also encompass linear DNA molecules. Specifically, the term "plasmid" also encompasses molecules obtained by, for example, cleaving a circular plasmid with restriction endonucleases, thereby converting the circular plasmid molecule into a linear forms, as well as linear molecules capable of replication in prokaryotes. Plasmids can replicate, i.e., amplify independently of the genomic genetic information stored in the nucleoid of prokaryotic cells, and can be used for cloning, i.e., amplifying genetic information in bacterial cells. For example, the DNA plasmid of the present application is a plasmid constructed based on the pGEMHE plasmid.

This application provides a recombinant virus comprising any of the nucleic acid molecules or vectors mentioned above.

In some embodiments, the recombinant virus is an adeno-associated virus (AAV), chimeric AAV, adenovirus, retrovirus, lentivirus, herpes simplex virus, baculovirus, or any mutant or derivative thereof. Preferably, the recombinant virus is AAV. In some embodiments, the AAV comprises one or more of the following: AAV1, AAV2, AAV3, AAV4, AAV5, AAV6, AAV7, AAV8, AAV9, AAV10, AAV11, AAV12, AAVrh8, AAVrh10, AAVrh36, AAVrh37, AAVrh74, AAVrh79, AAV-DJ, AAV-DJ/8, AAV.Anc80, AAV.Anc80L65, AAV-PHP.B, AAV-PHP.B2, AAV-PHP.B3, AAV-PHP.A, AAV-PHP.eB, AAV-PHP.S, AAV2i8, MyoAAV, AAVMYO, and AAV.CPP.16 capsid serotype or a variant thereof.

### Pharmaceutical composition

This application provides a pharmaceutical composition comprising any of the aforementioned light-sensitive channel proteins, nucleic acid molecules, vectors or recombinant viruses, and a pharmaceutically acceptable carrier.

The form of a pharmaceutical composition depends on multiple criteria, including, for example, route of administration, disease severity, or dosage.

In some embodiments, the pharmaceutical composition may be formulated for delivery to a subject via appropriate routes, including but not limited to oral administration, injection (such as intravenous injection, intramuscular injection, subcutaneous injection, intradermal injection, intracardiac injection, intrathecal injection, intrapleural injection, intraperitoneal injection, etc.), mucosal administration (such as intranasal administration, buccal administration, etc.), sublingual administration, rectal administration, transdermal administration, intraocular administration, or pulmonary administration. According to the intended administration route, the pharmaceutical composition can be prepared as tablet, capsule, pill, sugar-coated pill, powder, granule, cachet, lozenge, suppository, suspension, emulsion, syrup, aerosol (as solid or in liquid media), spray, ointment, paste, patch, cream, lotion, gel, inhalant, etc.

Those skilled in the art can understand that the dosage and frequency of administration of pharmaceutical composition can vary depending on the age, weight, individual response to vaccines, and the specific administration chosen.

### Therapeutic methods and applications

This application provides use of the above-mentioned light-sensitive channel proteins for treating retinal photoreceptor cell degenerative diseases.

This application provides use of the above-mentioned light-sensitive channel protein in the preparation of a medicament for treating retinal photoreceptor cell degenerative diseases.

This application provides a method for treating retinal photoreceptor cell degenerative disease, comprising administering a therapeutically effective amount of the above-mentioned pharmaceutical composition to a subject.

Among them, in the above methods or uses, the retinal photoreceptor cell degenerative diseases can encompass various known retinal photoreceptor cell degenerative diseases in this field, such as retinitis pigmentosa (RP), age-related macular degeneration (AMD), cone-rod dystrophy, and Leber congenital amaurosis (LCA), etc.

It should be understood that this application includes various aspects, embodiments, and combinations of the aspects and/or embodiments described herein. The above description and subsequent examples are intended to illustrate rather than limit the scope of the present application. Other aspects, improvements, and modifications within the scope of this application will be apparent to those skilled in the art to which this application belongs. Therefore, ordinary technical personnel in this field should recognize that the scope of this application also includes the improvements and modifications to the aspects and examples.

### Examples

### Example 1: construction of VR2.0 series mutant expression plasmids based on engineered microbial photosensitive protein PsCatCh and their functional comparison at the level of Xenopus laevis oocyte

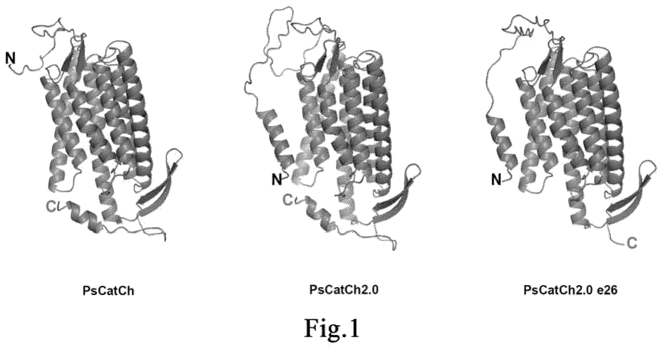

In order to enhance the properties of the photosensitive proteins, mainly to improve plasma membrane transporting efficiency and photosensitivity, based on previous research and understanding of the structure (Fig. 1) and function of PsCatCh 2.0 photosensitive protein, we performed amino acid truncation and mutagenesis on the PsCatCh portion (amino acid sequence is shown in SEQ ID NO.1) of PsCatCh 2.0. The PsCatCh 2.0 protein comprises seven transmembrane domains, with an N-terminal region of about 30 amino acids located outside the membrane and a C-terminal region of about 40 amino acids located outside the membrane.

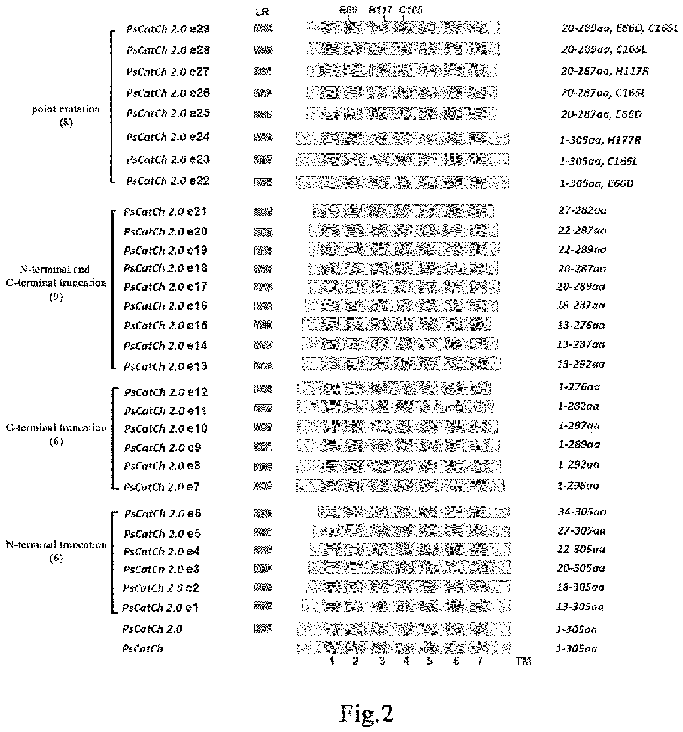

The specific truncation and mutation design schemes are shown in Fig. 2; Six N-terminal truncation variants of PsCatCh 2.0 were generated with deletions of 12, 17, 19, 21, 26, and 33 amino acids, named PsCatCh 2.0 (e1, e2, e3, e4, e5, or e6, respectively); additionally, six C-terminal truncation variants of PsCatCh 2.0 were constructed with deletions of 9, 13, 16, 18, 23, and 29 amino acids, named PsCatCh 2.0 (e7, e8, e9, e10, e11, or e12, respectively); furthermore, nine double-truncation variants were generated with combined N- and C- terminal truncations of PsCatCh 2.0: N12-C13 (12 amino acids truncated at the N-terminus, and 13 amino acids truncated at the C-terminus), N12-C18 (12 amino acids truncated at the N-terminus, and 18 amino acids truncated at the C-terminus), N12-C29 (12 amino acids truncated at the N-terminus, and 29 amino acids truncated at the C-terminus), N17-C18 (17 amino acids truncated at the N-terminus, and 18 amino acids truncated at the C-terminus), N19-C16 (19 amino acids truncated at the N-terminus, and 16 amino acids truncated at the C-terminus), N19-C18 (19 amino acids truncated at the N-terminus, and 18 amino acids truncated at the C-terminus), N21-C16 (21 amino acids truncated at the N-terminus, and 16 amino acids truncated at the C-terminus), N21-C18 (21 amino acids truncated at the N-terminus, and 18 amino acids truncated at the C-terminus), and N26-C22 (26 amino acids truncated from the N-terminus, 22 amino acids truncated from the C-terminus), named PsCatCh 2.0 (e13, e14, e15, e16, e17, e18, e19, e20, or e21, respectively).

In addition to the truncation strategy, we introduced single point mutations at specific amino acid residues (E66D, C165L, and H177R) in the ion channel functional region of both PsCatCh2.0 and its truncated variant PsCatCh2.0-e18, respectively, and these mutants were named PsCatCh2.0 (e22, e23, e24, e25, e26, or e27). PsCatCh2.0-e28 was obtained by substituting the N-terminal amino acid T with G and adding 2 amino acids LE to the C-terminus of PsCatCh2.0-e26; PsCatCh 2.0-e29 was derived from PsCatCh 2.0-e28 by introducing the E66D mutation, resulting in a double mutant (E66D and C165L).

We first constructed expression plasmids (with pGEMHE plasmid as the backbone) for the above photosensitive protein PsCatCh 2.0 (e1 to e29) mutant series using gene synthesis and molecular cloning methods. An LR signal peptide sequence (amino acid sequence is shown in SEQ ID NO.2) was added to the N-terminus of the photosensitive protein. To facilitate detection of the expressed photosensitive protein, the following sequences were added to the C-terminus of photosensitive protein: (1) a fused fluorescent protein YFP sequence (amino acid sequence is shown in SEQ ID NO.3), (2) a T sequence (amino acid sequence is shown in SEQ ID NO.4) to enhance cell membrane expression efficiency, and (3) an endoplasmic reticulum export signal sequence E (amino acid sequence SEQ ID NO.5). Specifically, the photosensitive protein VR2.0 was constructed in the following sequential order: the LR signal peptide sequence at the N-terminus, followed by the PsCatCh 2.0 amino acid sequence, and then the T sequence, and finally the E sequence. When fusing with a YFP fluorescent protein tag is required, YFP is inserted between the T sequence and E sequence. When the photosensitive protein VR2.0 is used for clinical gene therapy, it does not contain the YFP fluorescent protein.

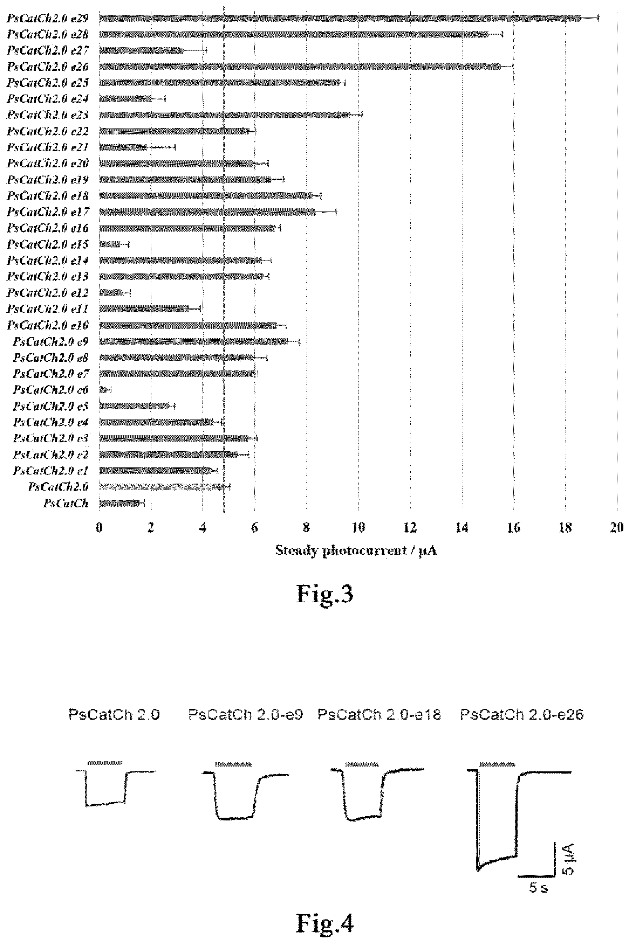

The plasmids constructed in this example have been validated by gene sequencing, confirming the accuracy of the plasmid sequences. Subsequently, RNA encoding the photosensitive protein was synthesized using the AmpliCap MaxT7 kit and microinjected into Xenopus laevis oocytes. The light-evoked responses in Xenopus laevis oocytes expressing the photosensitive protein were recorded using a dual electrode voltage clamp. Specifically, Xenopus laevis oocytes were cultured in ND96 solution supplemented with 1 µM all-trans-retinal at a temperature of 16°C. In order to block the opening of endogenous chloride ion channels in cells activated by Ca²⁺, 50 nl of the Ca²⁺ chelator BAPTA (200mM) was microinjected into Xenopus laevis oocytes. The BAPTA-injected Xenopus laevis oocytes were incubated at 16°C for 90 minutes. Then, dual-electrode voltage-clamp recordings were performed at room temperature of 25°C. Under light stimulation (450nm wavelength, 5mW/mm², -60mV), stable photocurrent responses were detected for the photosensitive protein PsCatCh 2.0 e-series mutants, as shown in Fig. 3 and Table 1.

**Table 1**

| **Name of photosensitive protein** | **PsCat Ch2.0** | **e1** | **e2** | **e3** | **e4** | **e5** | **e6** | **e7** | **e8** | **e9** | **e10** |
|---|---|---|---|---|---|---|---|---|---|---|---|
| Average photocurrent value (µA) | 4.83 | 4.34 | 5.36 | 5.74 | 4.41 | 2.69 | 0.28 | 6.03 | 5.95 | 7.27 | 6.85 |
| Relative change rate to PsCatCh2.0 (%) | 0.00 | -10.14 | 10.97 | 18.84 | -8.70 | -44.31 | -94.2 0 | 24.84 | 23.19 | 50.52 | 41.8 2 |

| **Name of photosensitive protein** | **PsCat Ch2.0** | **e11** | **e12** | **e13** | **el4** | **e15** | **e16** | **el7** | **e18** | **e19** | **e20** |
|---|---|---|---|---|---|---|---|---|---|---|---|
| Average photocurrent value | 4.83 | 3.46 | 0.94 | 6.35 | 6.27 | 0.81 | 6.80 | 8.33 | 8.23 | 6.62 | 5.92 |
| Relative change rate to PsCatCh2.0 (%) | 0.00 | -28.36 | -80.5 4 | 31.47 | 29.81 | -83.23 | 40.79 | 72.46 | 70.39 | 37.06 | 22.5 7 |

| **Name of photosensitive protein** | **PsCat Ch2.0** | **e21** | **e22** | **e23** | **e24** | **e25** | **e26** | **e27** | **e28** | **e29** | |
|---|---|---|---|---|---|---|---|---|---|---|---|
| Average photocurrent value | 4.83 | 1.85 | 5.80 | 9.68 | 2.03 | 9.28 | 15.49 | 3.25 | 15.03 | 18.59 | |
| Relative change rate to PsCatCh2.0 (%) | 0.00 | -61.70 | 20.08 | 100.4 1 | -57.9 7 | 92.18 | 220.7 5 | -32.7 4 | 211.18 | 284.8 9 | |

Among them, the current of PsCatCh is about 1.55 µA, and the current of PsCatCh 2.0 is about 4.83 µA. The currents of PsCatCh 2.0-e1, PsCatCh 2.0-e18, PsCatCh 2.0-e26, PsCatCh 2.0-e28, and PsCatCh 2.0-e29 are about 4.34 µA, 8.23 µA, 15.49 µA, 15.03 µA, and 18.59 µA, respectively. Among the variants, 10 exhibited reduced photocurrents compared to PsCatCh 2.0, while19 demonstrated improved photocurrent. The research results indicate that appropriate N-terminal or (and) C-terminal truncation can effectively enhance the photosensitivity of the photosensitive protein PsCatCh 2.0.

For the point mutation strategy, the E66D mutation on the PsCatCh 2.0 backbone has an approximately 20% increase in photocurrent; the C165L mutation has an approximately 100.4% increase in photocurrent; the H117R mutation results in a decrease of approximately 58%. Similar trends in magnitude were observed for point mutations introduced into the PsCatCh 2.0-e18. The photocurrent of PsCatCh 2.0-e29 with E66D and C165L double mutations was approximately 3.56 µA higher than that of PsCatCh 2.0-e28 containing only C165L single mutation. These results demonstrate a synergistic effect between the truncation strategy and point mutation strategy for engineering the photosensitive protein PsCatCh. We compared the photocurrents of representative photosensitive proteins PsCatCh2.0-e9, PsCatCh2.0-e18, and PsCatCh2.0-e26 with PsCatCh2.0, and the results are shown in Fig. 4.

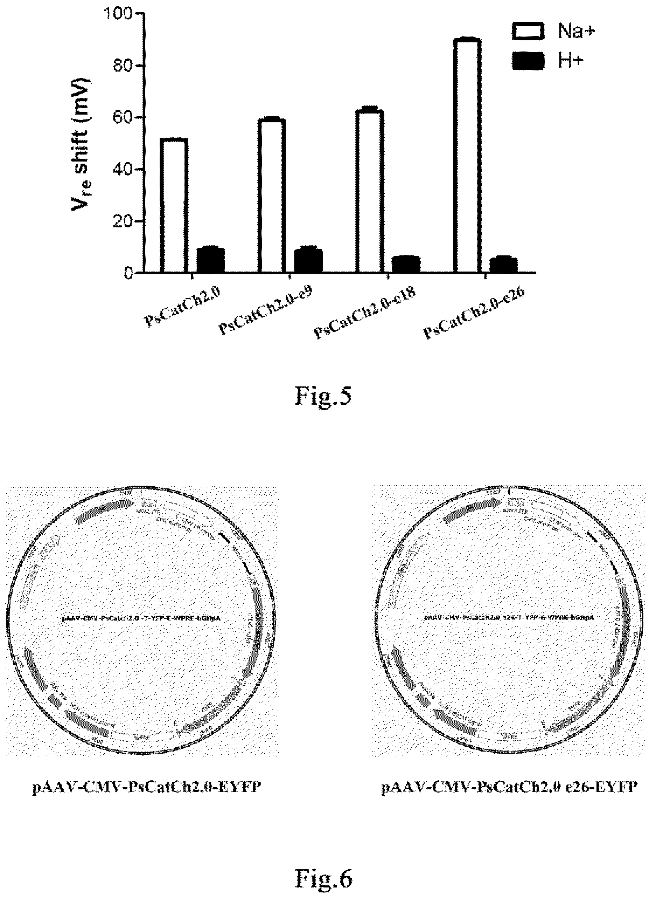

As shown in Fig. 5, regarding ion selectivity, PsCatCh2.0-e9, PsCatCh2.0-e18, and PsCatCh2.0-e26 exhibited varying degrees of increased sodium ion (Na⁺) permeability and slightly decreased hydrogen ion (H⁺) permeability compared to PsCatCh2.0, which is beneficial for mitigating the adverse effects caused by excessive intracellular acidification resulting from H⁺ ions accumulation.

In summary, we designed multiple mutants using the strategy of amino acid truncation and point mutation. After in vitro cell level testing and screening, we obtained multiple VR2.0 series photosensitive protein mutants with varying levels of improved photoresponse properties.

### Example 2: Construction of core plasmid vectors expressing photosensitive proteins and preparation of rAAVs

After preliminary *in vitro* screening of multiple mutants exhibiting superior photosensitivity compared to PsCatCh2.0, three photosensitive protein mutants PsCatCh2.0-e9, PsCatCh2.0-e18, and PsCatCh2.0-e26 were selected to construct core plasmid vectors and packaged into rAAVs, followed by subsequent validation and functional characterization.

To enable efficient expression of the photosensitive proteins in retinal cells, the broad-spectrum CMV promoter was employed. For convenient detection of the expressed photosensitive protein, the following genetic elements were incorporated at its C-terminus: (1) a fused YFP sequence, (2) a T sequence to enhance cell membrane expression efficiency, (3) an endoplasmic reticulum export signal sequence E. WPRE elements (nucleotide sequence is shown in SEQ ID NO.6) and HGHpA sequence (nucleotide sequence is shown in SEQ ID NO.7) were also added. The pAAV-MCS plasmid backbone was utilized to construct the pAAV-CMV-VR2.0-T-EYFP-E-WPRE-HGHpA core plasmid series through molecular cloning and gene synthesis techniques, as shown in Fig. 6. Sequencing verification results confirmed the correct construction of all plasmids.

The type 2 AAV serotype highly efficient for infecting retinal cells was selected for intravitreal injection. Recombinant AAV (rAAV) was packaged in HEK293 cells using the triple-plasmid transfection system, comprising the pAAV-RC2 serotype plasmid, pAAV-AdHelper helper plasmid, and pAAV-CMV-VR2.0-T-EYFP-E-WPRE-HGHpA core plasmid. After 72 hours of transfection, cell pellet and culture supernatants were collected. The rAAVs were then obtained by iodixanol (idox) ultracentrifugation purification, viral titers were determined in vg/ml, and aliquots were stored in a -80°C refrigerator.

### Example 3: photoresponses of HEK293T cells expressing VR2.0 series recorded with patch clamp

The preferred core plasmid carrying the expression cassette of the photosensitive protein VR2.0 series was transfected into adherently cultured HEK293T cells. After transfection, the cells were further incubated for 48 hours, followed by whole-cell voltage-clamp recordings under constant room temperature (25°C). The main experimental conditions were as follows: the extracellular solution consisted of 140mM sodium chloride, 5mM potassium chloride, 2mM calcium chloride, 20mM 4-hydroxyethylpiperazine ethanesulfonic acid, and 16mM glucose, wherein the pH was adjusted to 7.4 with sodium hydroxide, and the solution was placed at room temperature; the intracellular solution consisted of 115mM cesium methylsulfonate, 20mM cesium chloride, 2.5mM magnesium chloride, 0.6mM ethylene glycol bis (2-aminoethyl ether) tetraacetic acid, 10mM 4-hydroxyethylpiperazine ethanesulfonic acid, 4mM adenosine 5'-triphosphate magnesium salt, 0.4mM guanosine 5'- triphosphate sodium salt, and 10mM phosphocreatine, wherein the pH was adjusted to 7.2 with cesium hydroxide, and the solution was placed on ice. The extracellular solution was pre-oxygenated with 100% O₂ 30 minutes before the experiment. A P-1000 horizontal puller (Sutter Instrument Co.) was used to pull glass microelectrodes with resistances of 6-8 MΩ. The HEK293T cells to be tested were dark-adapted in the extracellular solution for 30 minutes to stabilize their physiological state before patch-clamp recordings. To verify the photosensitivity of the target photosensitive protein, the photocurrents were recorded at a light intensity of 1.66× 10¹⁵ photons/cm²s, and the open time constant and closed time constant were analyzed using Clampfit 10.6 software. In addition, to investigate the light response frequency of the tested photosensitive protein, pulsed light stimuli at 2, 4, 8, 16, and 32 Hz were applied. The light source was a Mightex external optical fiber, with stimulation timing controlled by BioLED software, and the specific light intensity measured by an optical power meter.

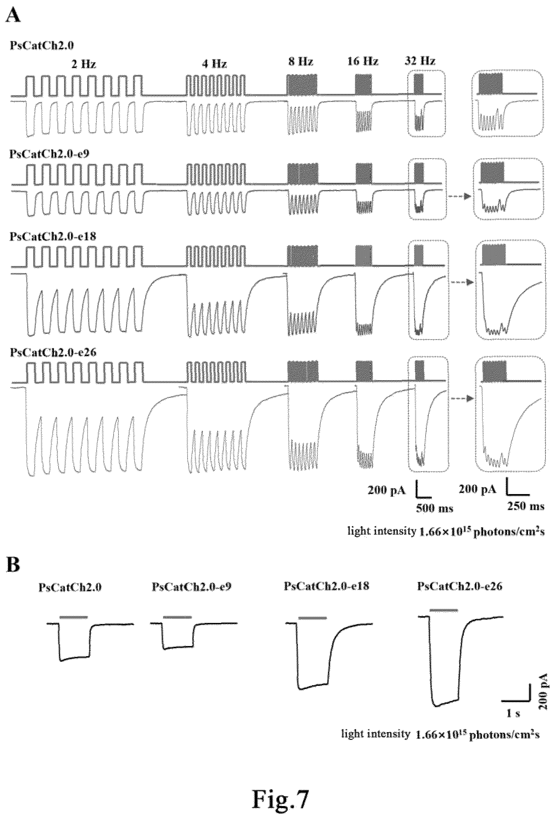

The experimental results are shown in Fig. 7. From Fig. 7A, we can see that VR2.0 maintained robust photocurrent responses to light stimulation at a high frequency of 32Hz under 470 nm wavelength illumination with an intensity of 1.66×10¹⁵ photons/cm²s. From Fig. 7B further demonstrates that under 470 nm wavelength illumination with an intensity of 1.66×10¹⁵ photons/cm²s, some VR2.0 mutants showed varying degrees of improvement in the current generated after 1 second of stimulation compared to PsCatCh2.0.

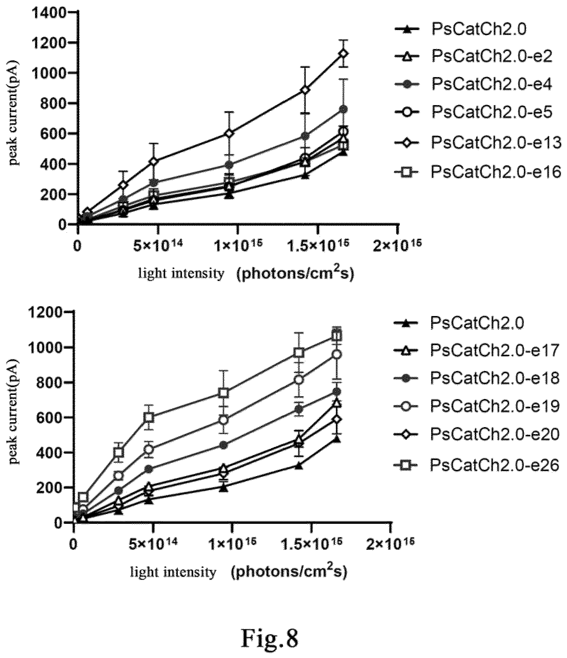

We also tested the photocurrent responses of some mutants of the VR2.0 series under different light intensity conditions at 470nm wavelength, and the results are shown in Fig. 8. The results show that multiple photosensitive protein mutants exhibit favorable photocurrent responses, showing an upward trend with elevated light intensity. Among them, PsCatCh2.0-e2 , PsCatCh2.0-e4 , PsCatCh2.0-e5 ; PsCatCh2.0-e13 , PsCatCh2.0-e16 , PsCatCh2.0-e17, PsCatCh2.0-e18, PsCatCh2.0-e19, PsCatCh2.0-e20, and PsCatCh2.0-e26 all displayed enhanced performance compared to PsCatCh2.0.

As illustrated in Fig. 8, photocurrent signals of approximately 50.00 pA and 130.00 pA were generated by PsCatCh2.0-e18 and PsCatCh2.0-e26, respectively, under a light intensity of 7.9×10¹³ photons/cm²s, which are 2.22-fold and 5.78-fold higher than the 22.50 pA photocurrent produced by PsCatCh2.0, respectively.

According to the guidelines of the International Commission on Non Ionizing Radiation Protection (ICIRP), the light intensity applied to the retina must not exceed the safety threshold for the corresponding wavelength, and visual restoration requires high spatiotemporal resolution. These two critical characteristics necessitate photosensitive proteins simultaneously exhibit high photosensitivity for retinal safety and fast kinetics to meet the demands of high spatiotemporal resolution. Therefore, the safe light intensity of 470nm blue light for the retina must not exceed 7.62×10¹⁴ photons/cm²s. In this example, some photosensitive protein VR2.0 mutants we used generated significant photocurrent at 5.92×10¹³ photons/cm²s, which is much lower than the photocurrent generated under the safe light intensity threshold conditions for the retina and does not have phototoxic effects on retinal cells. In terms of response frequency, while visual signal processing requires 24Hz, the photosensitive protein VR2.0 exhibited responsiveness to light stimulation up to at least 32Hz, thereby fully meeting the requirements of visual signal processing.

### Example 4: intravitreal injection of rAAVs in rd1 mice

For the animal experiments, postnatal 4-week-old retinal pigment degeneration model mice *(rdl* mice) and wild-type C57BL/6J mice were selected. The mice were anesthetized via intraperitoneal injection with a mixture of 100mg/kg ketamine and 12mg/kg xylazine based on body weight. After achieving sufficient anesthesia, the ocular surface and periorbital skin were disinfected with 0.5% povidone-iodine. To minimize discomfort during intravitreal injection, topical anesthesia was administered to the mouse eyes using proparacaine hydrochloride eye drops (Alcaine). The mice were immobilized and the eyeballs were exposed. 1.5 µL of rAAV2-CMV-VR2.0 virus (titer: approximately 2.5E+12vg/mL) was taken using a Nanoject III high-precision microinjector equipped with a glass microelectrode. The injection was performed intravitreally by inserting the needle 0.5mm below the corneal limbus on the nasal side of the mouse, and the contralateral eye was injected in the same manner. A control group receiving only the injection vehicle was established in parallel. After the injection, levofloxacin hydrochloride ophthalmic gel (Jie Qi) was applied to the mouse eyeballs to prevent infection. One month post-injection, therapeutic efficacy assessment and behavioral observations were conducted in *rd1* mice.

### Example 5: recording the flash visual evoked potential of RGCs (Retinal ganglion cells) expressing the VR2.0 series in mouse retina

Wild type C57BL/6J mice (positive control group), *rdl* mice treated with intravitreal injection of rAAV2-CMV-VR2.0 (experimental group), and untreated littermate *rd1* mice (negative control group) were anesthetized via intraperitoneal injection of a mixture of 100mg/kg ketamine and 12mg/kg xylazine. The fur between both eyes and ears of the mouse was removed using a hair clipper to fully expose the bregma and the tip of the Lambdoid sutures. The mouse head was immobilized using a brain stereotaxic device (RWD, Shenzhen, China). 48 hours before the FVEP experiment, a silver wire electrode with a diameter of 0.25mm was implanted into the right primary visual cortex (recording electrode, 3.6mm away from the bregma point and 2.3mm lateral on both sides). Before the experiment, the mice were dark-adapted for 8 hours, followed by intraperitoneal anesthesia. Compound tropicamide eye drops (0.5% tropicamide+0.5% phenylephrine hydrochloride) were used to dilate the pupils for 5 minutes. The reference electrode was inserted subcutaneously between the eyes, and the ground electrode was clamped on the tail of the mouse. 64 repetitions of light stimuli (2800 µs, blue light, 5.0 cds/m²) were performed using a flash stimulator (IRC, Chongqing, China). With a band-pass filter set at 3.0 - 70.0Hz, experimental results were recorded at at a sampling rate of 2000Hz. The Flash visual evoked potential (FVEP) data was generated and recorded using RetiMINIR 4.0 software, and the N1 amplitude data table was obtained.

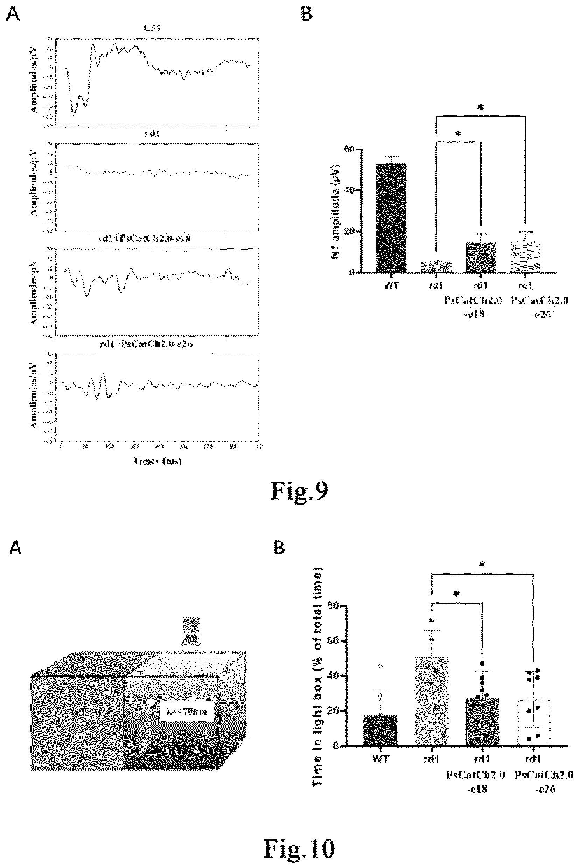

The experimental results are shown in Fig. 9. The results indicate that: (1) the FVEP N1-wave amplitude in wild-type C57BL/6J mice injected with PBS solvent was 53.03 µV, n=3; (2) the FVEP N1-wave amplitude in *rd1* mice injected with PBS solvent was 3.14 µV, n=4; (3) the FVEP N1-wave amplitude in *rd1* mice treated with intravitreal injection of rAAV2-CMV-PsCatCh2.0-e18 virus was 12.37 µV, n=4; and (4) the FVEP N1-wave amplitude in *rd1* mice treated with intravitreal injection of rAAV2-CMV-PsCatCh2.0-e26 virus was 14.81 µV, n=4.

The above experimental results indicate that visual signals generated in the retina of *rd1* mice following intravitreal injection of rAAV2-CMV-VR2.0 were successfully transmitted to the V1 region of the visual cortex.

### Example 6: light induced light/dark box behavior experiment in mice

The light/dark box consists of two equally sized compartments (18cm × 20cm × 18cm) on both sides, connected by an arched doorway (7cm × 5cm). The light box was equipped with a LED light source of 470nm wavelength (Mightex, Canada), while the dark box was wrapped with a black cloth cover. All experimental mice were aged 10-12 weeks and dard-adapted for 2 hours before the experiment. All behavioral experiments were conducted between 18:00 and 21:00. At the beginning of the experiment, C57BL/6J mice, *rd1* mice treated with intravitreal injection of rAAV2-CMV-VR2.0, and untreated *rd1* mice were individually placed in the bright box with blue light (wavelength of 470nm, light intensity of 4.7×10¹⁴ photons/cm²s), and allowed to explore freely. Mouse head position was analyzed to determine their movement patterns between light and dark boxes. Subsequently, the collected data was imported into GraphPad Prism 7 software and evaluated for significance using one-way ANOVA, with P<0.05 indicating statistically significant.

The ratio of time spent in the light box to the total time was analyzed, and the experimental results are shown in Fig. 10. The results show the following: (1) wild-type C57BL/6J mice injected with the vehicle (positive control): 17.29%, n = 7; (2) *rd1* mice treated with vehicle (negative control): 51.20%, n=5; (3) the group of *rd1* mice treated with intravitreal injection of rAAV2-CMV-PsCatCh2.0-e18 virus: 27.63%, n=8; (4) the group of *rd1* mice treated with intravitreal injection of rAAV2-CMV-PsCatCh2.0-e26 virus : 26.75%, n=6. These findings indicate that intravitreal injection of rAAV2-CMV-VR2.0 restored photophobic responses in *rd1* mice, proving compelling evidence that VR2.0 can effectively rescue visoin-guided behavior in *rd1* mice with retinal degeneration and its effectiveness in treating retinal degenerative disorders.

### Example 7: mouse optomotor response behavior experiment

4 Lenovo monitors (L1900pA) were used to display moving gratings, with a central activity platform (height: 17.5cm) for mice, and a mirror surface on the bottom. The grating parameters were programmed using Matlab, with each experiment lasting 12 minutes. In each phase, the grating rotated clockwise for 30 seconds, counterclockwise for 30 seconds, followed by a 10s pause. The angular velocity of the grating rotation was 12 °/s. The spatial frequency of the gratings at each stage (i.e., the number of grating bars within 1°, in cycles/degree (c/d)) was sequentially set as follows: 0.20 (for software and system testing), 0.10, 0.15, 0.20, 0.25, 0.30, 0.35, 0.40, 0.45, 0.50, and 0.60 c/d. Before the experiment, the mice were dark-adapted for 12 hours. C57BL/6J mice, *rd1* mice treated with intravitreal injection of rAAV2-CMV-VR2.0, and *rd1* mice were individually placed on an activity platform, and the visual acuity was evaluated using the optomotor system. Subsequently, the collected data was imported into GraphPad Prism 7 software to generate bar graphs. Statistical significance was evaluated using Student's t-test, with P<0.05 indicating statistically significant.

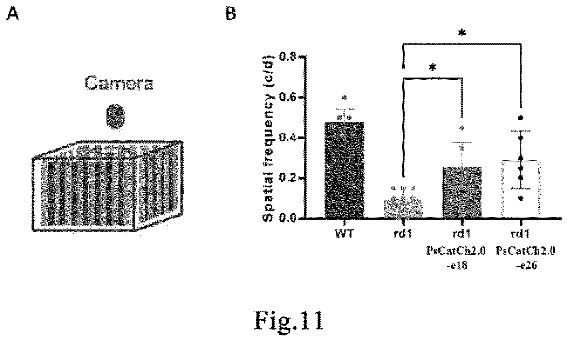

The experimental results are shown in Fig. 11. The results indicate that the average maximum visual acuity of the wild-type C57BL/6J mice injected with solvent (positive control) is 0.48 c/d, n=7; the maximum average visual acuity of the *rd1* mouse injection solvent (negative control) is 0.09 c/d, n=8; the maximum visual acuity of *rd1* mice treated with intravitreal injection of rAAV2-CMV-PsCatCh2.0-e18 virus is 0.25 c/d, n=6; and the maximum visual acuity of *rd1* mice treated with intravitreal injection of rAAV2-CMV-PsCatCh2.0-e26 virus is 0.29 c/d, n=6.

Following intravitreal injection of rAAV2-CMV-VR2.0, *rd1* mice significantly restores their light sensitivity, demonstrating that VR2.0 can restore the visually guided behavior in retinal degenerative *rd1* mice, confirming its therapeutic efficacy in treating retinal degeneration.

The sequences involved in the above examples are listed in Table 2.

**Table 2: Relevant sequence listing**

| Sequence number | Sequence description | Sequence |
|---|---|---|
| SEQ ID NO.1 | PsCatCh sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCFVDKEEEDEDERISTKTYANRALE |
| SEQ ID NO.2 | LR signal peptide sequence | MRPQILLLLALLTLGLANGTEGPNFYVPFSNKTGVVRS |
| SEQ ID NO.3 | Fluorescent protein YFP sequence | MVSKGEELFTGVVPILVELDGDVNGHKFSVSGEGEGDATYGKLTL KFICTTGKLPVPWPTLVTTFGYGLQCFARYPDHMKQHDFFKSAMP EGYVQERTIFFKDDGNYKTRAEVKFEGDTLVNRIELKGIDFKEDG NILGHKLEYNYNSHNVYIMADKQKNGIKVNFKIRHNIEDGSVQLA DHYQQNTPIGDGPVLLPDNHYLSYQSALSKDPNEKRDHMVLLEF VTAAGITLGMDELYK |
| SEQ ID NO.4 | T peptide sequence | KSRITSEGEYIPLDQIDINV |
| SEQ ID NO.5 | E peptide sequence | FCYENEV |
| SEQ ID NO.6 | WPRE sequence | AATCAACCTCTGGATTACAAAATTTGTGAAAGATTGACTGGTAT TCTTAACTATGTTGCTCCTTTTACGCTATGTGGATACGCTGCTTTA ATGCCTTTGTATCATGCTATTGCTTCCCGTATGGCTTTCATTTTCT CCTCCTTGTATAAATCCTGGTTGCTGTCTCTTTATGAGGAGTTGT GGCCCGTTGTCAGGCAACGTGGCGTGGTGTGCACTGTGTTTGC TGACGCAACCCCCACTGGTTGGGGCATTGCCACCACCTGTCAG CTCCTTTCCGGGACTTTCGCTTTCCCCCTCCCTATTGCCACGGCG GAACTCATCGCCGCCTGCCTTGCCCGCTGCTGGACAGGGGCTC GGCTGTTGGGCACTGACAATTCCGTGGTGTTGTCGGGGAAATCA TCGTCCTTTCCTTGGCTGCTCGCCTATGTTGCCACCTGGATTCTG CGCGGGACGTCCTTCTGCTACGTCCCTTCGGCCCTCAATCCAGC GGACCTTCCTTCCCGCGGCCTGCTGCCGGCTCTGCGGCCTCTTC CGCGTCTTCGCCTTCGCCCTCAGACGAGTCGGATCTCCCTTTGG GCCGCCTCCCCGC |
| SEQ ID NO.7 | HGHpA sequence | GGGTGGCATCCCTGTGACCCCTCCCCAGTGCCTCTCCTGGCCCT GGAAGTTGCCACTCCAGTGCCCACCAGCCTTGTCCTAATAAAAT TAAGTTGCATCATTTTGTCTGACTAGGTGTCCTTCTATAATATTAT GGGGTGGAGGGGGGTGGTATGGAGCAAGGGGCAAGTTGGGAA GACAACCTGTAGGGCCTGCGGGGTCTATTGGGAACCAAGCTGG AGTGCAGTGGCACAATCTTGGCTCACTGCAATCTCCGCCTCCTG GGTTCAAGCGATTCTCCTGCCTCAGCCTCCCGAGTTGTTGGGAT TCCAGGCATGCATGACCAGGCTCAGCTAATTTTTGTTTTTTTGGT AGAGACGGGGTTTCACCATATTGGCCAGGCTGGTCTCCAACTCC TAATCTCAGGTGATCTACCCACCTTGGCCTCCCAAATTGCTGGG ATTACAGGCGTGAACCACTGCTCCCTTCCCTGTCCTT |
| SEQ ID NO.8 | PsCatCh2.0-e1 sequence | TILLDDCTPIYLNVGPLWEQKVARGTQWFGVILSLAFLIYYIWITY KATCGWEELYVCTIEFCKIVIELYFEFSPPAMIYQTNGEVTPWLRYA EWLLTCPVICIHLSNITGLNDDYSGRTMSLITSDLGGICMAVTSALS KGWLKWLFFVIGCCYGASTFYHAALIYIESYYTMPHGVCKNMVL AMAAVFFTSWFMFPGLFLAGPEGTNALSWAGSTIGHTVADLLSKN AWGMIGHFLRLEIHKHIIIHGDVRRPITVNTLGREVTVSCFVDKEE EDEDERISTKTYANRALE |
| SEQ ID NO.9 | PsCatCh2.0-e2 sequence | DCTPIYLNVGPLWEQKVARGTQWFGVILSLAFLIYYIWITYKATCG WEELYVCTIEFCKIVIELYFEFSPPAMIYQTNGEVTPWLRYAEWLLT CPVICIHLSNITGLNDDYSGRTMSLITSDLGGICMAVTSALSKGWL KWLFFVIGCCYGASTFYHAALIYIESYYTMPHGVCKNMVLAMAA VFFTSWFMFPGLFLAGPEGTNALSWAGSTIGHTVADLLSKNAWG MIGHFLRLEIHKHIIIHGDVRRPITVNTLGREVTVSCFVDKEEEDED ERISTKTYANRALE |
| SEQ ID NO.10 | PsCatCh 2.0 e3 sequence | TPIYLNVGPLWEQKVARGTQWFGVILSLAFLIYYIWITYKATCGW EELYVCTIEFCKIVIELYFEFSPPAMIYQTNGEVTPWLRYAEWLLTC PVICIHLSNITGLNDDYSGRTMSLITSDLGGICMAVTSALSKGWLK WLFFVIGCCYGASTFYHAALIYIESYYTMPHGVCKNMVLAMAAV FFTSWFMFPGLFLAGPEGTNALSWAGSTIGHTVADLLSKNAWGMI GHFLRLEIHKHIIIHGDVRRPITVNTLGREVTVSCFVDKEEEDEDER ISTKTYANRALE |
| SEQ ID NO.11 | PsCatCh2.0-e4 sequence | IYLNVGPLWEQKVARGTQWFGVILSLAFLIYYIWITYKATCGWEEL YVCTIEFCKIVIELYFEFSPPAMIYQTNGEVTPWLRYAEWLLTCPVI CIHLSNITGLNDDYSGRTMSLITSDLGGICMAVTSALSKGWLKWL FFVIGCCYGASTFYHAALIYIESYYTMPHGVCKNMVLAMAAVFFT SWFMFPGLFLAGPEGTNALSWAGSTIGHTVADLLSKNAWGMIGH FLRLEIHKHIIIHGDVRRPITVNTLGREVTVSCFVDKEEEDEDERIST KTYANRALE |
| SEQ ID NO.12 | PsCatCh2.0-e5 sequence | GPLWEQKVARGTQWFGVILSLAFLIYYIWITYKATCGWEELYVCTI EFCKIVIELYFEFSPPAMIYQTNGEVTPWLRYAEWLLTCPVICIHLS NITGLNDDYSGRTMSLITSDLGGICMAVTSALSKGWLKWLFFVIG CCYGASTFYHAALIYIESYYTMPHGVCKNMVLAMAAVFFTSWFM FPGLFLAGPEGTNALSWAGSTIGHTVADLLSKNAWGMIGHFLRLEI HKHIIIHGDVRRPITVNTLGREVTVSCFVDKEEEDEDERISTKTYAN RALE |
| SEQ ID NO.13 | PsCatCh2.0-e6 sequence | VARGTQWFGVILSLAFLIYYIWITYKATCGWEELYVCTIEFCKIVIE LYFEFSPPAMIYQTNGEVTPWLRYAEWLLTCPVICIHLSNITGLNDD YSGRTMSLITSDLGGICMAVTSALSKGWLKWLFFVIGCCYGASTF YHAALIYIESYYTMPHGVCKNMVLAMAAVFFTSWFMFPGLFLAG PEGTNALSWAGSTIGHTVADLLSKNAWGMIGHFLRLEIHKHIIIHG DVRRPITVNTLGREVTVSCFVDKEEEDEDERISTKTYANRALE |
| SEQ ID NO.14 | PsCatCh 2.0 e7 sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCFVDKEEEDEDERIST |
| SEQ ID NO.15 | PsCatCh2.0-e8 sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCFVDKEEEDEDE |
| SEQ ID NO.16 | PsCatCh2.0-e9 sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCFVDKEEED |
| SEQ ID NO.17 | PsCatCh2.0-e10 sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCFVDKEE |
| SEQ ID NO.18 | PsCatCh2.0-e11 sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCF |
| SEQ ID NO.19 | PsCatCh2.0-e12 sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG RE |
| SEQ ID NO.20 | PsCatCh2.0-e13 sequence | TILLDDCTPIYLNVGPLWEQKVARGTQWFGVILSLAFLIYYIWITY KATCGWEELYVCTIEFCKIVIELYFEFSPPAMIYQTNGEVTPWLRYA EWLLTCPVICIHLSNITGLNDDYSGRTMSLITSDLGGICMAVTSALS KGWLKWLFFVIGCCYGASTFYHAALIYIESYYTMPHGVCKNMVL AMAAVFFTSWFMFPGLFLAGPEGTNALSWAGSTIGHTVADLLSKN AWGMIGHFLRLEIHKHIIIHGDVRRPITVNTLGREVTVSCFVDKEE EDEDE |
| SEQ ID NO.21 | PsCatCh2.0-e14 sequence | TILLDDCTPIYLNVGPLWEQKVARGTQWFGVILSLAFLIYYIWITY KATCGWEELYVCTIEFCKIVIELYFEFSPPAMIYQTNGEVTPWLRYA EWLLTCPVICIHLSNITGLNDDYSGRTMSLITSDLGGICMAVTSALS KGWLKWLFFVIGCCYGASTFYHAALIYIESYYTMPHGVCKNMVL AMAAVFFTSWFMFPGLFLAGPEGTNALSWAGSTIGHTVADLLSKN AWGMIGHFLRLEIHKHIIIHGDVRRPITVNTLGREVTVSCFVDKEE |
| SEQ ID NO.22 | PsCatCh2.0-e15 sequence | TILLDDCTPIYLNVGPLWEQKVARGTQWFGVILSLAFLIYYIWITY KATCGWEELYVCTIEFCKIVIELYFEFSPPAMIYQTNGEVTPWLRYA EWLLTCPVICIHLSNITGLNDDYSGRTMSLITSDLGGICMAVTSALS KGWLKWLFFVIGCCYGASTFYHAALIYIESYYTMPHGVCKNMVL AMAAVFFTSWFMFPGLFLAGPEGTNALSWAGSTIGHTVADLLSKN AWGMIGHFLRLEIHKHIIIHGDVRRPITVNTLGRE |
| SEQ ID NO.23 | PsCatCh2.0-e16 sequence | DCTPIYLNVGPLWEQKVARGTQWFGVILSLAFLIYYIWITYKATCG WEELYVCTIEFCKIVIELYFEFSPPAMIYQTNGEVTPWLRYAEWLLT CPVICIHLSNITGLNDDYSGRTMSLITSDLGGICMAVTSALSKGWL KWLFFVIGCCYGASTFYHAALIYIESYYTMPHGVCKNMVLAMAA VFFTSWFMFPGLFLAGPEGTNALSWAGSTIGHTVADLLSKNAWG MIGHFLRLEIHKHIIIHGDVRRPITVNTLGREVTVSCFVDKEE |
| SEQ ID NO.24 | PsCatCh2.0-e17 sequence | TPIYLNVGPLWEQKVARGTQWFGVILSLAFLIYYIWITYKATCGW EELYVCTIEFCKIVIELYFEFSPPAMIYQTNGEVTPWLRYAEWLLTC PVICIHLSNITGLNDDYSGRTMSLITSDLGGICMAVTSALSKGWLK WLFFVIGCCYGASTFYHAALIYIESYYTMPHGVCKNMVLAMAAV FFTSWFMFPGLFLAGPEGTNALSWAGSTIGHTVADLLSKNAWGMI GHFLRLEIHKHIIIHGDVRRPITVNTLGREVTVSCFVDKEEED |
| SEQ ID NO.25 | PsCatCh2.0-e18 sequence | TPIYLNVGPLWEQKVARGTQWFGVILSLAFLIYYIWITYKATCGW EELYVCTIEFCKIVIELYFEFSPPAMIYQTNGEVTPWLRYAEWLLTC PVICIHLSNITGLNDDYSGRTMSLITSDLGGICMAVTSALSKGWLK WLFFVIGCCYGASTFYHAALIYIESYYTMPHGVCKNMVLAMAAV FFTSWFMFPGLFLAGPEGTNALSWAGSTIGHTVADLLSKNAWGMI GHFLRLEIHKHIIIHGDVRRPITVNTLGREVTVSCFVDKEE |
| SEQ ID NO.26 | PsCatCh2.0-e19 sequence | IYLNVGPLWEQKVARGTQWFGVILSLAFLIYYIWITYKATCGWEEL YVCTIEFCKIVIELYFEFSPPAMIYQTNGEVTPWLRYAEWLLTCPVI CIHLSNITGLNDDYSGRTMSLITSDLGGICMAVTSALSKGWLKWL FFVIGCCYGASTFYHAALIYIESYYTMPHGVCKNMVLAMAAVFFT SWFMFPGLFLAGPEGTNALSWAGSTIGHTVADLLSKNAWGMIGH FLRLEIHKHIIIHGDVRRPITVNTLGREVTVSCFVDKEEED |
| SEQ ID NO.27 | PsCatCh2.0-e20 sequence | IYLNVGPLWEQKVARGTQWFGVILSLAFLIYYIWITYKATCGWEEL YVCTIEFCKIVIELYFEFSPPAMIYQTNGEVTPWLRYAEWLLTCPVI CIHLSNITGLNDDYSGRTMSLITSDLGGICMAVTSALSKGWLKWL FFVIGCCYGASTFYHAALIYIESYYTMPHGVCKNMVLAMAAVFFT SWFMFPGLFLAGPEGTNALSWAGSTIGHTVADLLSKNAWGMIGH FLRLEIHKHIIIHGDVRRPITVNTLGREVTVSCFVDKEE |
| SEQ ID NO.28 | PsCatCh2.0-e21 sequence | GPLWEQKVARGTQWFGVILSLAFLIYYIWITYKATCGWEELYVCTI EFCKIVIELYFEFSPPAMIYQTNGEVTPWLRYAEWLLTCPVICIHLS NITGLNDDYSGRTMSLITSDLGGICMAVTSALSKGWLKWLFFVIG CCYGASTFYHAALIYIESYYTMPHGVCKNMVLAMAAVFFTSWFM FPGLFLAGPEGTNALSWAGSTIGHTVADLLSKNAWGMIGHFLRLEI HKHIIIHGDVRRPITVNTLGREVTVSCF |
| SEQ ID NO.29 | PsCatCh2.0-e22 sequence | MGFQLNPEYLNETILLDDCTPIYLNVGPLWEQKVARGTQWFGVIL SLAFLIYYIWITYKATCGWEDLYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCFVDKEEEDEDERISTKTYANRALE |
| SEQ ID NO.30 | PsCatCh2.0-e23 sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIHLSNITGLNDDYSGRTMSLITSD LGGICMAVTSALSKGWLKWLFFVIGLCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCFVDKEEEDEDERISTKTYANRALE |
| SEQ ID NO.31 | PsCatCh2.0-e24 sequence | MGFQLNPEYLNETILLDDCTPIYLNVGPLWEQKVARGTQWFGVIL SLAFLIYYIWITYKATCGWEELYVCTIEFCKIVIELYFEFSPPAMIYQ TNGEVTPWLRYAEWLLTCPVICIRLSNITGLNDDYSGRTMSLITSD LGGICMAVTSALSKGWLKWLFFVIGCCYGASTFYHAALIYIESYY TMPHGVCKNMVLAMAAVFFTSWFMFPGLFLAGPEGTNALSWAG STIGHTVADLLSKNAWGMIGHFLRLEIHKHIIIHGDVRRPITVNTLG REVTVSCFVDKEEEDEDERISTKTYANRALE |
| SEQ ID NO.32 | PsCatCh2.0-e25 sequence | TPIYLNVGPLWEQKVARGTQWFGVILSLAFLIYYIWITYKATCGW DELYVCTIEFCKIVIELYFEFSPPAMIYQTNGEVTPWLRYAEWLLTC PVICIHLSNITGLNDDYSGRTMSLITSDLGGICMAVTSALSKGWLK WLFFVIGCCYGASTFYHAALIYIESYYTMPHGVCKNMVLAMAAV FFTSWFMFPGLFLAGPEGTNALSWAGSTIGHTVADLLSKNAWGMI GHFLRLEIHKHIIIHGDVRRPITVNTLGREVTVSCFVDKEE |
| SEQ ID NO.33 | PsCatCh2.0-e26 sequence | TPIYLNVGPLWEQKVARGTQWFGVILSLAFLIYYIWITYKATCGW EELYVCTIEFCKIVIELYFEFSPPAMIYQTNGEVTPWLRYAEWLLTC PVICIHLSNITGLNDDYSGRTMSLITSDLGGICMAVTSALSKGWLK WLFFVIGLCYGASTFYHAALIYIESYYTMPHGVCKNMVLAMAAV FFTSWFMFPGLFLAGPEGTNALSWAGSTIGHTVADLLSKNAWGMI GHFLRLEIHKHIIIHGDVRRPITVNTLGREVTVSCFVDKEE |
| SEQ ID NO.34 | PsCatCh2.0-e27 sequence | TPIYLNVGPLWEQKVARGTQWFGVILSLAFLIYYIWITYKATCGW EELYVCTIEFCKIVIELYFEFSPPAMIYQTNGEVTPWLRYAEWLLTC PVICIRLSNITGLNDDYSGRTMSLITSDLGGICMAVTSALSKGWLK WLFFVIGCCYGASTFYHAALIYIESYYTMPHGVCKNMVLAMAAV FFTSWFMFPGLFLAGPEGTNALSWAGSTIGHTVADLLSKNAWGMI GHFLRLEIHKHIIIHGDVRRPITVNTLGREVTVSCFVDKEE |
| SEQ ID NO.35 | PsCatCh2.0-e28 sequence | GPIYLNVGPLWEQKVARGTQWFGVILSLAFLIYYIWITYKATCGW EELYVCTIEFCKIVIELYFEFSPPAMIYQTNGEVTPWLRYAEWLLTC PVICIHLSNITGLNDDYSGRTMSLITSDLGGICMAVTSALSKGWLK WLFFVIGLCYGASTFYHAALIYIESYYTMPHGVCKNMVLAMAAV FFTSWFMFPGLFLAGPEGTNALSWAGSTIGHTVADLLSKNAWGMI GHFLRLEIHKHIIIHGDVRRPITVNTLGREVTVSCFVDKEELE |
| SEQ ID NO.36 | PsCatCh2.0-e29 sequence | GPIYLNVGPLWEQKVARGTQWFGVILSLAFLIYYIWITYKATCGW DELYVCTIEFCKIVIELYFEFSPPAMIYQTNGEVTPWLRYAEWLLTC PVICIHLSNITGLNDDYSGRTMSLITSDLGGICMAVTSALSKGWLK WLFFVIGLCYGASTFYHAALIYIESYYTMPHGVCKNMVLAMAAV FFTSWFMFPGLFLAGPEGTNALSWAGSTIGHTVADLLSKNAWGMI GHFLRLEIHKHIIIHGDVRRPITVNTLGREVTVSCFVDKEELE |
| SEQ ID NO.37 | Linker peptide sequence | VDTSSRSR |

Among them, the amino acid sequences of the aforementioned PsCatCh2.0-e1 to PsCatCh2.0-e29 only include the PsCatch variant part, and do not include the LR signal peptide, T peptide, and E peptide parts.

## Claims

1. A light-sensitive channel protein comprising any one of the following PsCatCh variants:
(1) a protein obtained by truncating 1-33 amino acids at the N-terminus of the reference protein shown in SEQ ID NO.1;
(2) a protein obtained by truncating 1-29 amino acids at the C-terminus of the reference protein shown in SEQ ID NO.1;
(3) a protein obtained by mutations introduced around the retinal binding site of the reference protein shown in SEQ ID NO.1;
(4) a combination of any two or three of the above (1), (2), and (3); or
(5) a protein having more than 90%, 91%, 92%, 93%, 94%, 95%, 96%, 97%, 98%, or 99% sequence identity with the proteins described in (1), (2), (3), or (4).

2. The light-sensitive channel protein according to claim 1, wherein the light-sensitive channel protein comprises a protein obtained by truncating 12-26 amino acids at the N-terminus of the reference protein shown in SEQ ID NO.1.

3. The light-sensitive channel protein according to claim 2, wherein the light-sensitive channel protein comprises a protein obtained by truncating 17-21 amino acids at the N-terminus of the reference protein shown in SEQ ID NO.1.

4. The light-sensitive channel protein according to any one of claims 1-3, wherein the light-sensitive channel protein comprises a protein obtained by truncating 9-23 amino acids at the C-terminus of the reference protein shown in SEQ ID NO.1.

5. The light-sensitive channel protein according to claim 4, wherein the light-sensitive channel protein comprises a protein obtained by truncating 9-18 amino acids at the C-terminus of the reference protein shown in SEQ ID NO.1.

6. The light-sensitive channel protein according to claim 1, wherein the light-sensitive channel protein comprises a protein obtained by truncating 12-21 amino acids at the N-terminus and 13-23 amino acids at the C-terminus of the reference protein shown in SEQ ID NO.1.

7. The light-sensitive channel protein according to any one of claims 1-6, wherein the light-sensitive channel protein comprises a protein obtained by introducing the following mutations into the reference protein shown in SEQ ID NO.1 or the PsCatCh variant thereof: substitution of E at position 66 with D, and/or substitution of C at position 165 with L.

8. The light-sensitive channel protein according to any one of claims 1-7, wherein the amino acid sequence of the light-sensitive channel protein is shown in SEQ ID NO.8, SEQ ID NO.9, SEQ ID NO.13, SEQ ID NO.14, SEQ ID NO.15, SEQ ID NO.16, SEQ ID NO.19, SEQ ID NO.20, SEQ ID NO.22, SEQ ID NO.23, SEQ ID NO.24, SEQ ID NO.25, SEQ ID NO.26, SEQ ID NO.28, SEQ ID NO.29, SEQ ID NO.31, SEQ ID NO.32, SEQ ID NO.33, SEQ ID NO.34, SEQ ID NO.35, or SEQ ID NO.36, or
has more than 90%, 91%, 92%, 93%, 94%, 95%, 96%, 97%, 98%, or 99% sequence identity with SEQ ID NO.8, SEQ ID NO.9, SEQ ID NO.13, SEQ ID NO.14, SEQ ID NO.15, SEQ ID NO.16, SEQ ID NO.19, SEQ ID NO.20, SEQ ID NO.22, SEQ ID NO.23, SEQ ID NO.24, SEQ ID NO.25, SEQ ID NO.26, SEQ ID NO.28, SEQ ID NO.29, SEQ ID NO.31, SEQ ID NO.32, SEQ ID NO.33, SEQ ID NO.34, SEQ ID NO.35, or SEQ ID NO.36.

9. The light-sensitive channel protein according to any one of claims 1-8, wherein the light-sensitive channel protein further comprises an LR signal peptide fused to the N-terminus of the PsCatCh variant, and/or
a T peptide linked to the C-terminus of the PsCatCh variant and a E peptide linked to the T peptide,
wherein the amino acid sequence of the LR signal peptide is shown in SEQ ID NO.2, the amino acid sequence of the T peptide is shown in SEQ ID NO.4, and the amino acid sequence of the E peptide is shown in SEQ ID NO.5.

10. A nucleic acid molecule comprising a nucleotide sequence encoding the light-sensitive channel protein according to any one of claims 1-9.

11. A vector comprising the nucleic acid molecule according to claim 10.

12. A recombinant virus comprising the nucleic acid molecule according to claim 10 or the vector according to claim 11.

13. The recombinant virus according to claim 12, wherein the recombinant virus is a recombinant adeno-associated virus.

14. A pharmaceutical composition comprising the light-sensitive channel protein according to any one of claims 1-9, the nucleic acid molecule according to claim 10, the vector according to claim 11, or the recombinant virus according to claim 12 or 13, and a pharmaceutically acceptable carrier.

15. Use of the light-sensitive channel protein according to any one of claims 1-9 in the preparation of a medicament for treating retinal photoreceptor cell degenerative diseases.

16. The use according to claim 15, wherein the retinal photoreceptor cell degenerative diseases comprise retinitis pigmentosa (RP), age-related macular degeneration (AMD), cone-rod dystrophy, and Leber congenital amaurosis (LCA).
